# EUROPEAN PATENT APPLICATION

(11) **EP 0 966 935 A1**
(43) Date of publication of application: **29.12.1999**
(21) Application number: 98124493.2
(22) Date of filing: 28.12.1998
(51) Int. Cl.: A61F 5/451, A61F 5/443, A61F 5/441

(54) **Urine collector**

(30) Priority: 26.06.1998 WO PCT/US98/13289; 26.06.1998 WO PCT/US98/13288
(71) Applicant: THE PROCTER & GAMBLE COMPANY, Cincinnati, Ohio 45202 (US)
(72) Inventor: D'Acchioli, Vincenzo, 65779 Kelkheim am Taunus (DE); Palumbo, Gianfranco, 61348 Bad Homburg (DE)
(74) Representative: Kohol, Sonia

(57) **Abstract**

The present invention relates to a urine management device (10). It comprises a bag (11) having an aperture (13), and an anatomically-shaped flange (12), which surrounds the aperture (13). The flange (12) provides for adhesive attachment to the uro-genital of the wearer. In particular, the bag (11) comprises a wall material having an inner surface (18) and an outer surface (18), with the outer surface (18) being provided with a hydrophobic fibrous layer, preferably a nonwoven layer. In another aspect of the present invention, the urine management device (10) is used in combination with a disposable diaper.

## Description

### Field of the Invention

The present invention relates to a urine management device having a softer tactile feel for increased wearer comfort and satisfaction.

### Background of the Invention

Urine management devices are known articles of manufacture that are designed to be worn principally by incontinence sufferers and infants. Such urine management devices are attached to the uro-genital region of the wearer and are intended to entrap and immediately contain urine and other bodily discharges. As a consequence, these devices are functionally effective in lessening epidermal irritation; in preventing contamination of articles such as clothing and bedding; and even in preventing the soiling of the carers themselves. Nevertheless, a problem often encountered during the use of such urine management devices is that the constituent material of the outer surface of the devices tends to cause discomfort and in some cases, extreme discomfort to, for example, the bedridden wearer or to the infant.

Typically, the urine management devices are made from a plastic material. For instance, GB 1,092,274 discloses a pediatric urine collector for female use comprising a collector bag of plastic material opening. The base of the opening is provided with a wedge like projection adapted to engage the lower perineal area of the infant. The collector is secured to the body of the wear by adhesive material. GB 2,268,882 discloses a urostomy pouch/bag of plastic material provided with a stomal orifice which is surrounded by a first coupling member, by which the pouch can be releasably affixed to a counterpart coupling member. The pouch may also be provided for use as a kit further comprising an applicator of super absorbent material which can be injected into the pouch by the use of a plunger. EP 140 478 discloses a disposable diaper having a water proof barrier preferably polypropylene or polyethylene formed as a flattened bag having a single opening. The bag is provided with 2 layers filler materials, the upper layer being a wicking material and the lower being a super absorbent material. WO 85/03428 discloses a urine retaining device for male patients comprising a protecting bag having an opening, parallelogram-walls of a water impermeable laminate of polyethylene film and nonwoven provided with an insert of water absorbing material.

However plastic material, while functionally acceptable, is endowed with certain characteristics that are unsatisfactory and disagreeable to the wearer. The feel or texture of plastic material is particularly disturbing from the point of view of skin healthiness. Typically, the skin of incontinence sufferers and infants is especially sensitive and needs to be treated gently and with care. It has been recognised that the rubbing of plastic material from a urine management device against the body of a wearer during wear can lead to occlusion, create hot clammy conditions and stickiness, reddening, skin rashes and perhaps, even lead to a more severe skin irritation and ultimately to the disregarding of the device. In addition the use of plastic material tends to make audible noise when the wearer moves, which may cause embarrassment. Therefore, a real consumer need can be identified for a urine management device with superior cushioning qualities and a softer tactile feel.

The present invention addresses this need by providing the outer surface of the urine management device with a hydrophobic fibrous layer, which is preferably a nonwoven layer. It has been found that the presence of this fibrous hydrophobic layer is uniquely advantageous and greatly enhances the softness of the urine management device without adversely affecting its functionality. Furthermore, the urine management device is aesthetically more pleasing, produces less crinkle during use and results in a high level of wearer and carer satisfaction in relation to skin healthiness.

In another aspect of the present invention, the urine management device with its hydrophobic fibrous outer surface outer layer can be advantageously used with a disposable diaper. In particular the presence of the hydrophobic fibrous outer layer ensures that any fluid which may contact the outer surface is repelled and is hence absorbed by the diaper and is not absorbed by the outer layer itself.

### Summary of the Invention

A urine management device (10) constructed in accordance with the present invention comprises a bag (11) said bag having an aperture (13) and a flange (12) surrounding the aperture (13) for releasable adhesive attachment to the uro-genital area of the wearer. The flange is attached to the bag. The bag (11) comprises a wall material that has an outer surface (17) and an inner surface (18). The outer surface (30) is provided with a hydrophobic fibrous layer, wherein said fibres have a wetting time of a least 1 hour. Preferably said layer is a nonwoven layer. The inner surface (18) preferably comprises a plastic film layer. The plastic film is preferably permeable to air and to vapour.

In a preferred embodiment of the present invention, the hydrophobic fibrous layer (17), which is preferably a nonwoven layer and the plastic film layer (18) are in the form of a laminate.

In another aspect of the present invention, the urine management device is used in combination with a disposable diaper.

### Brief description of the drawings

It is believed that the invention will be better understood from the foregoing description in conjunction with the accompanying drawings in which:
Figure 1 is a plan view of a disposable urine management device of the present invention.
Figure 2 is a side view of the disposable urine management device of Figure 1.
Figure 3 is a cross-sectional view taken along line 3-3 of Figure 1.
Figure 4 is a plan view of another embodiment of a disposable urine management device of the present invention.
Figure 5 is a cross-sectional view taken along line 5-5 of Figure 4.
Figure 6 is a cross-sectional view of another embodiment of a disposable urine management device of the present invention.

### Detailed description of the Invention

The term "disposable" as used herein describes devices which generally are not intended to be laundered or otherwise restored or reused (i.e., they are intended to be discarded after a single use and, preferably, to be recycled, composted or otherwise disposed of in an environmentally compatible manner).

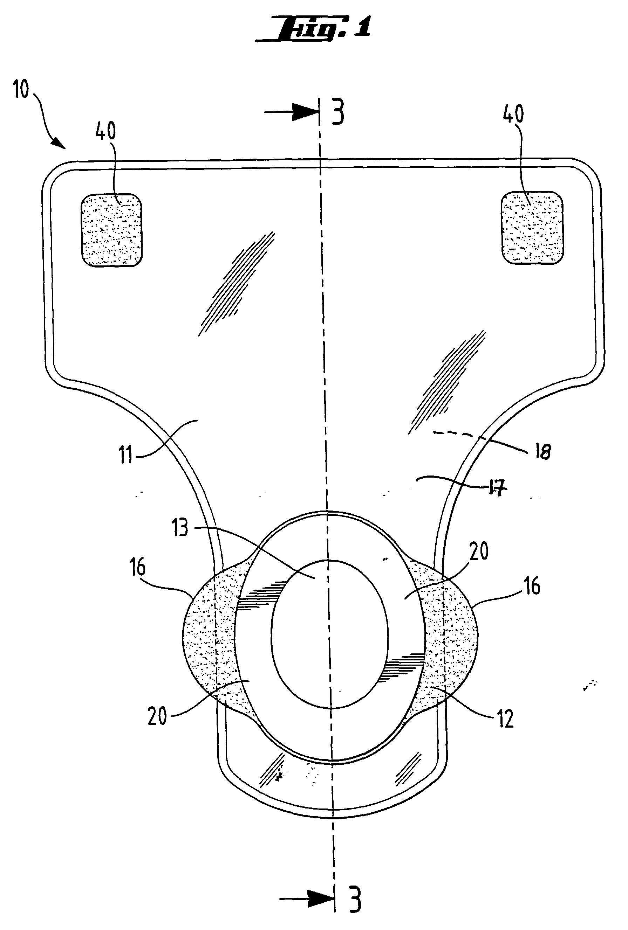

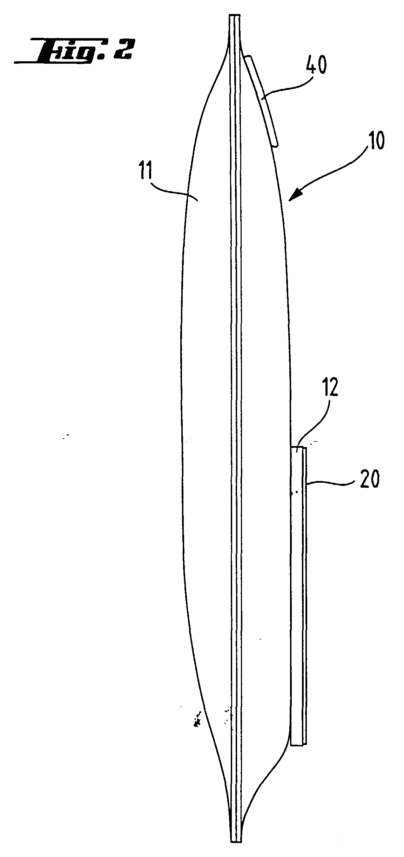

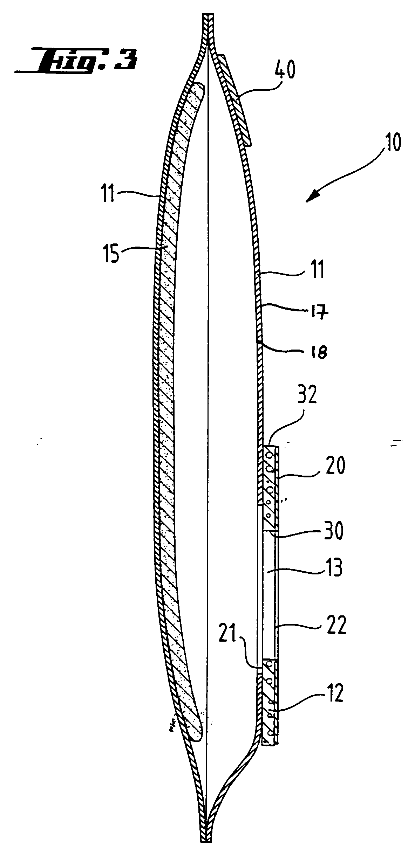

Referring now to figures 1 - 3, there is shown a urine management device (10). Typically disposable urine management devices (10) comprise a bag (11) having an aperture (13) and a flange (12) surrounding the aperture (13) for preferably adhesive attachment to the uro-genital area of a wearer. Any urine management device (10) known in the art can be provided according to the present invention.

The bag (11) as used herein is a flexible receptacle for the containment of discharged urine matter. The bag (11) can be provided in any shape or size depending on the intended use thereof, i.e. whether the device is intended for bedridden patients or active patients suffering from incontinence or for infants. For example elongated bags which are principally tubular or rectangular are typically utilised by bedridden patients and elderly incontinence sufferers. For more active wearers whether infants or adults, the urine management device should preferably be anatomically shaped such that the device follows the contours of the body and can be worn inconspicuously by the wearer under normal garments.

Particularly, preferred shapes are fiat circular and flat T shaped type bags, cone shaped bags, truncated cone shaped bags and pyramidal or truncated pyramidal shaped bags. In a most preferred embodiment of the present invention, the bag (11) has a substantially flat T shape.

In addition, the bag (11) is preferably shaped to fit the uro-genital region of the wearer and ensure good contact between the flange and the skin of the wearer. For example the bag (11) may be provided with a neck portion or conduit.

The bag (11) is preferably designed to provide sufficient volume for urine under a variety of wearing conditions, also when worn by a freely moving, i.e. not bedridden wearer.

The bag (11) is designed to safely contain any entrapped material, typically it will be liquid impermeable, yet it may be breathable. The bag (11) is designed of sufficient strength to withstand rupture in use, also when pressure on the bag (11) is exerted.

According to the present invention, depending on the shape of the bag (11) required, the bag (11) may be provided from a unitary piece of material or from a number of separate pieces of material, which may be identical or different and which are sealed at their respective peripheries.

According to the present invention the bag (11) can comprise one or multiple layers, preferably two or three layers. The layer on the inside of the bag, which will typically at least partially come in contact with urine and or other bodily excretions is called the inner layer (18). The outermost layer of the bag, which will typically at least partially come in contact with the skin to the wearer and the garments of the wearer, is called the outer layer (17).

According to the present invention an essential feature of the bag material is that the outer layer (17) of the bag is a fibrous layer, wherein the fibres are hydrophobic such that said fibres have a wetting time of at least 1 hour, preferably at least 6 hours and most preferably at least 24 hours. It has been surprisingly found that the provision of such a hydrophobic fibrous outer layer provides the bag with particularly pleasing sensation to the skin and in particular when utilised in conjunction with a diaper ensures that fluid is not absorbed by the outer layer of the wall material of the urine management device but is absorbed within the core of the diaper. In addition, the use of a fibrous outer layer reduces the audible noise often associated with plastic bag devices on movement of the wearer.

The hydrophobicity of the fibrous outer layer is determined according to the liquid absorbency time test as per the ASTM -D 1117-80 method.

According to the present invention any fibre which meets the hydrophobicity requirements can be usefully employed as the outer layer (17). Suitable fibres include fibres which meet the hydrophobicity requirements per se such as polyester fibres, polypropylene fibres, polyethylene fibres, polyamide fibres and mixtures thereof. Alternatively fibres which have been treated by the utilisation of a particular finishing treatment for example an oil, in order to provide them with the required hydrophobicity requirements may also be effectively utilised herein. Moreover mixtures of hydrophobic fibres or treated hydrophobic fibres may also be utilised. The fibrous outer layer may be provided as a woven or a nonwoven layer, preferably a nonwoven.

The additional layers of the bag material may be provided from any material, preferably so that the bag is liquid impervious. The layers may in particular comprise any material such as nonwovens or films. In a preferred embodiment of the present invention a laminate may be formed from a nonwoven layer and a film. The laminate can be formed by means known to the man skilled in the art.

Any nonwoven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose - wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., Ill, US under the designation EXXAIRE or those supplied by Mitsui Toatsu Co., Japan under the designation ESPOIR NO; and monolithic breathable materials such as Hytrel^{TM} available from DuPont and Pebax™ available from ELF Atochem, France.

In a preferred embodiment a film, which is comprised in any layer, is preferably permeable to gases such as air and to vapour such as water vapour in order to avoid the problem of entrapment and condensation of moisture vapour given off by the body of the wearer and thus, the hot, clammy and uncomfortable conditions after a short period of use.

The outer layer of the bag is preferably provided with a hydrophobic fibrous nonwoven layer. Such material layers present an uneven surface to the skin of the wearer and thus reduce significantly the problem of occlusion and greatly improves skin healthiness.

In one preferred embodiment of the present invention the bag comprises two layers. Preferably the outer layer (17) comprises said fibrous hydrophobic nonwoven layer and the inner layer (18) comprises a film.

In yet another preferred embodiment of the present invention, the bag (11) comprises three layers, preferably one film and two nonwoven layers. In an even more preferable embodiment the film is interposed between the two nonwoven layers. This sequence of layers results in a closed fibrous structure, which has a particularly pleasing sensation on contact with the skin of the wearer. In yet another preferred embodiment the inner layer comprises a film and the other two layers comprise nonwovens.

Typically, the nonwoven layer is treated with a surface active material, such as a fluorchemical or other hydrophobic finishings, to provide the requisite hydrophobicity. The nonwoven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The nonwoven layer can also be treated with agents to improve the tactile perceivable softness of the bag. The agents include but are not limited to vegetable, animal or synthetic oils, silicone oils and the like. The presence of these agents are known to impart a silky or flannel-like feel to the nonwoven layer without rendering it greasy or oily to the tactile sense of the wearer. Additionally, surfactant material, including anionic, non-anionic, cationic and non-cationic surfactants, may be added to further enhance softness and surface smoothness.

Furthermore, the nonwoven layer may be impregnated with a lotion to provide desirable therapeutic or protective coating lotion benefits. The lotion is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the nonwoven layer with a solid oil phase of cream formulation or to incorporate into the nonwoven layer an array of pressure- or thermal- or hydrorupturable capsules containing for example, baby oil.

In one embodiment of the present invention the bag (11) may contain absorbent material (15). The absorbent material (15) may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material (15) may be positioned in the bag (11) in any suitable manner. For example, the absorbent material (15) may be loosely arranged within the bag (11) or may be secured to the inner layer of the bag (11). Any known techniques for securing absorbent material (15) to nonwoven and film substrates may be used to secure the absorbent material to the inner layer of the bag (11). The absorbent material may also be arranged to have any desired shape or configuration (e.g., rectangular, oval, circular, etc.).

In the embodiment shown in Figures 1-3, the outer surface of bag (11) is provided with patches of adhesive (40) for securing the bag (11) to the body of the wearer. Preferably, the patches of adhesive (40) are positioned on the outer surface of bag (11) such that they are secured to the abdomen of the wearer in use. Any number, size and shape of adhesive patches (40) may be used depending on the intended use of the device. The adhesive (40) may be any medically approved water resistant pressure sensitive adhesive such as hydrocolloid adhesives and hydrogel adhesives. Particularly effective adhesives in providing the desired adhesive properties to secure the flange to the skin of the wearer whilst allowing for relatively painless application and removal are hydrophillic hydrogels formed from crosslinking polymers with a plastisicer to form a 3-dimensional matrix.

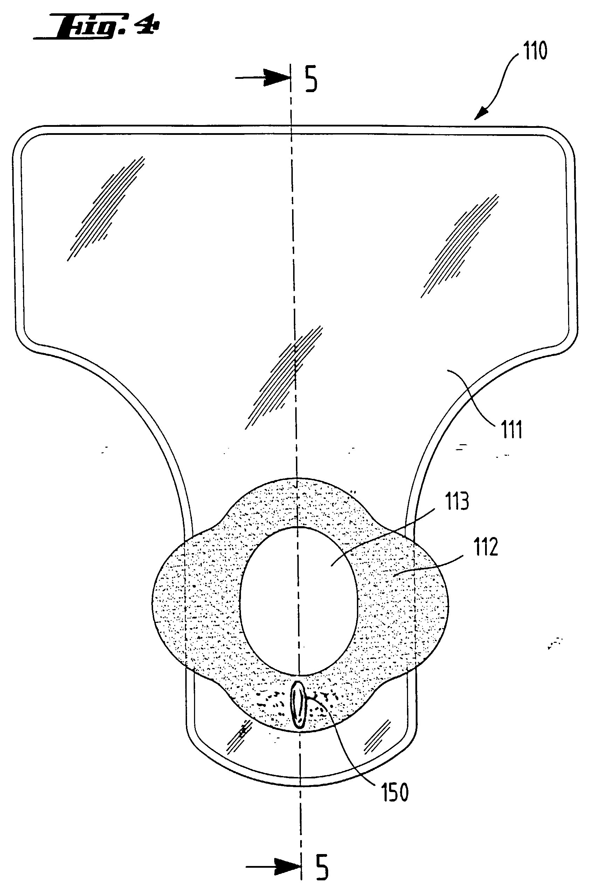

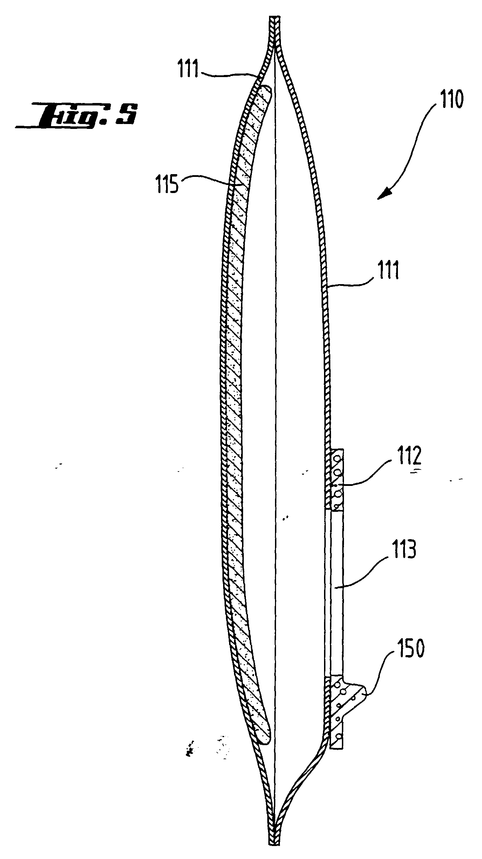

Referring now to Figures 4-5, there is shown another embodiment of a disposable urine management device (110). Disposable urine management device (110) comprises a bag (111) having an aperture (113), a flange (112) surrounding the aperture for adhesive attachment to the body of a wearer, and absorbent material (115) contained within the bag (111).

The flange (112) includes a raised, curved bulge (150) positioned beneath the aperture (113) and extending across the flange (112) for approximately the width of the aperture (113). The bulge (150) is shaped to span the perineum of the wearer.

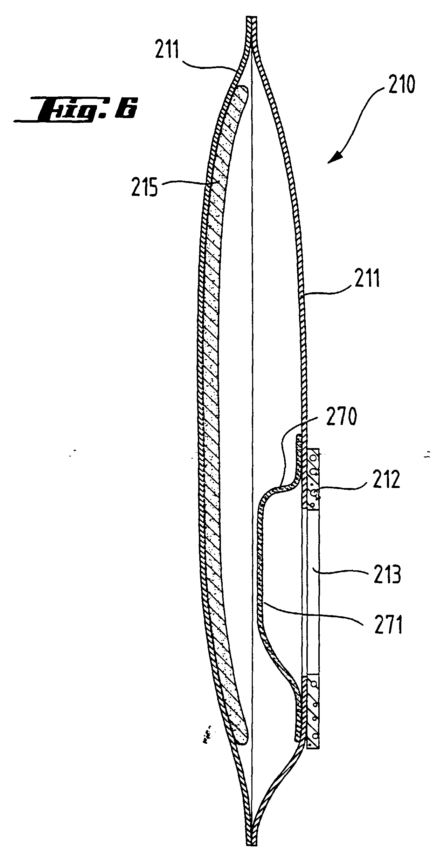

Referring now to Figure 6, there is shown another embodiment of a disposable urine management device (210). Disposable urine management device (210) comprises a bag (211) having an aperture (213), a flange (212) surrounding the aperture for adhesive attachment to the body of a wearer, and absorbent material (215) contained within the bag (211).

Disposable urine management device (210) also comprises an additional acquisition layer (270). Acquisition layer (270) is shown in Figure 6 to be secured to the inner surface of bag (211). However, the acquisition layer (270) may also be secured to the flange (212), or both the flange (212) and the inner surface of bag (211). Acquisition layer (270) is preferably positioned such that it separates the genitalia of the wearer from coming into direct contact with the absorbent material (215). Acquisition layer (270) is fluid pervious allowing urine to readily pass through so that it may be absorbed by absorbent material (215).

The acquisition layer (270) may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. If the acquisition, barrier layer includes fibers, the fibers may be spunbond, carded, wet-laid, meltblown, hydroentangled, or otherwise processed as is known in the art.

The acquisition layer (270) is designed to have a pore size such that the absorbent material (215) is not allowed to pass through and contact the wearer's skin. While designed not to have to large of a pore size which permits the passage of absorbent material (215), the acquisition layer (270) preferably has a pore size which is greater than the pore size of the absorbent material (215).

Preferably, the acquisition layer (270) is less hydrophilic than the absorbent material (215). The acquisition layer (270) may be treated with a surfactant to increase its initial wettability. When treated with surfactant, however, the acquisition layer (270) should still be less hydrophilic than the absorbent material (215). Suitable methods for treating the acquisition layer (270) with a surfactant include spraying the acquisition layer (270) with the surfactant and immersing the material into the surfactant. Alternatively, a surfactant may be incorporated into the acquisition layer (270).

As shown in Figure 1 the bag (11) is provided with an aperture (13) whereby urine is received from the body prior to storage within the bag cavity. The aperture (13) is surrounded by a flange (12) and may be provided in any shape or size, such as circular, oblong, heart shaped and may be symmetrical or asymmetrical, preferably the aperture has an oblong configuration either in the longitudinal or in the transversal direction, most preferably the contours of the aperture are in the shape of two ellipses with the respective main axes being substantially perpendicular.

The flange (12) is attached to the bag (11) according to any means known to the man skilled in the art which may provide permanent or releasable attachment. Preferably however, the flange (12) is attached to the bag (11) by adhesive. Typically, the bag (11) will be attached to the flange (12), towards the outer periphery of flange (12) so as not to cause any obstruction for the entering liquids.

The flange (12) may be provided in any size depending on the wearer group for which the device is intended. Similarly the flange (12) may be provided in any shape and preferably has a symmetrical shape preferably comprising a plurality of lobes (16).

The flange comprises a garment facing portion (21) and a wearer facing portion (22). In an preferred embodiment these are two large, substantially flat surfaces.

The flange (12) should be made of soft, flexible and malleable material to allow easy placement of the flange to the perianal area. In addition it is preferred that the flange (12) be made of a hydrophobic material such that if urine does come into contact with the perimeter (30) surrounding the aperture (13) it is repelled and does not wick to the outer edge (32) of the flange (12). Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a density of 5 to 250 g/m², more preferably 50 g/m². Other thermoplastic foam materials, or other suitable plastics sheet materials having the described properties of such foams (i.e., softness, pliability, stretchability, and contractability) might also be used. Preferably, the material of garment facing surface (21) of the flange (12) may extend into the defined aperture area so as to form a skirt or flap of material which prevents unintentional adhesion of the surface edges of the flange (12) defining the aperture (13) to oneanother during use.

According to the present invention the urine management device (10) further comprises an attachment means to secure the device to the wearer. Such means include straps and more preferably comprises a body-compatible pressure sensitive adhesive (20) applied to the wearer facing portion (22) of the flange (12).

The adhesive (20) is preferably covered with a release means (not shown) in order to protect the adhesive (20) such as siliconized paper. The adhesive (20) can cover the entire wearer facing surface of the flange or more preferably have at least one, preferably two to six non-adhesive portions. These portions may be adhesive free or may contain inactivated or covered adhesives. As is evident from Figure 1, the adhesive is in one preferred embodiment not applied to the entire wearer facing surface area of the flange (12), so as to provide lobes (16) on either side of the flange (12) which are non-adhesive and can thereby serve to facilitate placement and removal of the device whilst avoiding contact with the adhesive. These lobes are however preferably also covered by the release means. Before application of the urine management device (10) to the skin of the wearer, the release means if present is removed. Alternatively a single lobe placed centrally about the longitudinal axis of the flange (12) is also particularly beneficial.

According to the present invention any medically approved water resistant pressure sensitive adhesive may be used to attach the device to the uro-genital area of the wearer, such as hydrocolloid adhesives and hydrogel adhesives. Particularly effective adhesives in providing the desired adhesive properties to secure the flange to the skin of the wearer at the sensitive uro-genital area, whilst allowing for relatively painless application and removal, are formed from crosslinking polymers with a plastisicer to form a 3-dimensional matrix.

Suitable adhesives for use herein are hydrogel adhesives available from 3M and Promeon.

The adhesive (20) can be applied to the wearer facing surface (22) of the flange (12) by any means known in the art such as slot coating, spiral, or bead application or printing. Typically the adhesive (20) is applied at a basis weight of from 20g/m² to 2500g/m², more preferably from 500g/m² to 2000g/m² most preferably from 700g/m² to 1500g/m² depending on the end use envisioned. For example for urine management devices (10) to be used for babies the amount of adhesive (20) may be less than for urine management devices designed for active adult incontinence sufferers.

According to another aspect of the present invention, the urine management device (10) has been found particularly useful and beneficial when utilized in conjunction with a garment or a disposable diaper and/or a faecal management device. Typically the urine management device will be positioned to the uro-genital area of the wearer positioned and secured to the wearer by the adhesive flanges and patches of adhesives. Subsequently, the diaper is positioned over the urine management device (10) and fastened in a conventional manner around the body of the wearer. It has been found that in addition to providing excellent separation between urine and faecal matter, the combined urine management device (10) and diaper system reduce skin irritation, which may at time occur especially as the wearer group includes the very old, young and unhealthy wearers.

## Claims

1. A urine management device (10) comprising a bag (11), said bag (11) having an aperture (13) and a flange (12) surrounding said aperture (13) for releasable adhesive attachment to the uro-genital area of wearer, said flange (12) being attached to said bag (11), said bag (11) comprising a wall material, said wall material having an outer surface (17) and an inner surface (18) characterised in that said outer surface (17) is provided with a fibrous layer, wherein said fibres are hydrophobic and have a wetting time of at least 1 hour.

2. A urine management device according to claim 1, wherein said fibres have a wetting time of at least 6 hours.

3. A urine management device (10) according to claims 1 and 2, wherein said outer surface (17) comprises a nonwoven layer.

4. A urine management device (10) according to claim 1, wherein said inner surface (18) comprises a plastic film layer.

5. A urine management device (10) according to claim 4, wherein said plastic film layer is permeable to air and to vapour.

6. A urine management device (10) according to claims 3 and 4, wherein said nonwoven layer and said plastic film layer are in the form of a laminate.

7. A urine management device (10) according to claim 1, wherein said wall material comprises 3 layers, wherein said inner surface (18) is a nonwoven layer, said outer surface (17) is said fibrous hydrophobic layer and wherein a plastic film layer is interposed between said inner and said outer surface.

8. A urine management device (10) according to claim 7, wherein said inner surface nonwoven layer (18) is hydrophobic.

9. A urine management device (10) according to any of the preceding claims, wherein said hydrophobic fibrous outer layer (17) is softened with agents to enhance softness.

10. A urine management device (10) according to any of the preceding claims wherein said bag contains absorbent material (15) therein.

11. The use of a urine management device (10) according to any of the preceding claims in combination with a disposable diaper.
